Europäisches Patentamt

European Patent Office

Office européen des brevets

(11) Publication number: **0 174 319**
**B1**

(12) **EUROPEAN PATENT SPECIFICATION**

(45) Date of publication of patent specification: **22.06.88**

(51) Int. Cl.⁴: **F 28 D 7/10**

(21) Application number: **85900917.7**

(22) Date of filing: **25.01.85**

(86) International application number:
**PCT/US85/00127**

(87) International publication number:
**WO 85/04002 12.09.85 Gazette 85/20**

(54) **HEAT EXCHANGER FOR MASS TRANSFER DEVICE.**

(30) Priority: **27.02.84 US 583853**

(43) Date of publication of application:
**19.03.86 Bulletin 86/12**

(45) Publication of the grant of the patent:
**22.06.88 Bulletin 88/25**

(84) Designated Contracting States:
**BE DE FR GB SE**

(56) References cited:
**EP-A-0 108 525**
**DE-A-3 025 623**
**DE-A-3 146 089**
**FR-A- 552 370**
**FR-A-2 304 884**
**FR-A-2 525 476**
**GB-A- 684 602**
**US-A- 939 021**
**US-A-2 152 280**
**US-A-3 578 075**
**US-A-3 612 174**
**US-A-3 731 731**
**US-A-4 013 122**
**US-A-4 190 105**
**US-A-4 306 617**
**US-A-4 369 074**

(73) Proprietor: **BAXTER TRAVENOL LABORATORIES, INC.**
**One Baxter Parkway**
**Deerfield, IL 60015 (US)**

(72) Inventor: **LEONARD, Ronald, J.**
**19211 Crowley Road**
**Harvard, IL 60033 (US)**
Inventor: **JOHNSON, Kenneth, M.**
**1905 Burr Oak Lane**
**Lindenhurst, IL 60046 (US)**
Inventor: **DREHOBL, Thomas, L.**
**333 Highpoint Drive**
**Lindenhurst, IL 60046 (US)**

(74) Representative: **MacGregor, Gordon et al**
**ERIC POTTER & CLARKSON 14 Oxford Street**
**Nottingham, NG1 5BP (GB)**

Courier Press, Leamington Spa, England.

## Description

### Technical field

The present invention concerns a novel heat exchanger and a novel method for making the heat exchanger. The heat exchanger of the illustrative embodiment may be used with a mass transfer device, such as an oxygenator or a dialyzer, and it may be enclosed within the mass transfer device housing.

### Background art

Mass transfer devices, such as oxygenators and dialyzers, are known in which the mass transfer device housing encloses a heat exchanger for controlling the temperature of the blood. For example, a combination blood oxygenator and heat exchanger is disclosed in US—A—3,764,271. Further such devices are disclosed in US—A—3,731,731 and 2,152,280.

Although the heat exchanger of the present invention may be used in many different fields, and although no limitation is intended, for simplicity the heat exchanger of the present invention will be described herein as being used in a blood oxygenator. Disposable blood oxygenators are widely used today in connection with the oxygenation of a patient's blood, and the present invention is ideally suited for this field of use, among others.

It is desirable that a disposable blood oxygenator be relatively compact, have a low prime volume, be easy to manufacture, and have relatively low cost. It is important that a heat exchanger, used in connection with a disposable blood oxygenator, enable substantially uniform flow of the fluids that will be in heat exchange relationship with each other. It is also desirable that the oxygenator and heat exchanger be formed as a single unit, that the heat exchanger be positioned close to the oxygenating medium, such as the oxygenator fiber bundle, and that the heat exchanger have high efficiency with a relatively low pressure drop.

The prior art devices referred to above do not possess these characteristics to the extent desirable. The present invention provides a heat exchanger having the characteristics and stabilities set forth above.

### Disclosure of the invention

In accordance with the present invention, a heat exchanger is provided with comprises a core having an inside and an outside and formed of a corrugated metal having a high thermal conductivity. Each of the corrugations of the core comprises an outer wall, a contiguous first side wall, a contiguous inner wall, and a contiguous second side wall. The first and second side walls are substantially parallel to each other and a plurality of the corrugations repeat contiguously. A first fluid, such as blood, is introduced onto the outside of the core. A second fluid, such as water, is introduced into the inside of the core. The first and second fluids are in heat exchange relationship with each other and the flow of the fluids is substantially uniform resulting from the substantially parallel first, and second side walls of the corrugations, as known by the above-mentioned US—A—2 152 280. Inside the core there is a manifold, the fluid manifold defining a first longitudinal slot open to the outside of the fluid manifold and a second longitudinal slot open to the outside of the fluid manifold, with said first and second slots being on opposed sides of the manifold, said manifold having an intermediate wall which defines a first chamber communicating with the first slot and a second chamber communicating with the second slot, the fluid manifold being so configured that the second fluid may move in said first chamber, through said first slot, around the outside of the fluid manifold into said second slot and out from said second chamber.

In the illustrative embodiment, the core is formed by the steps of providing a flexible metal hose, annealing the flexible metal hose, compressing the annealed hose and expanding the compressed hose to a length that is substantially less than its original length out greater than its length during compression. These steps have been found to provide the substantial parallelism of the walls of the corrugations, enabling substantially uniform flow of the fluids.

In the illustrative embodiment, the second fluid flows through a manifold which is located within the inside of the core. The manifold comprises a generally S-shaped cross-sectional configuration. The S-shape is defined by an intermediate wall having opposed ends with a first curved member extending from one end toward the other end but spaced therefrom to form a first slot, and with the second curved member extending from the other end and toward the one end but spaced therefrom to form a second slot. The second fluid is introduced into the first chamber defined by one side of the intermediate wall and the first curved member. In this manner, the second fluid will exit from the second chamber defined by the opposite side of the intermediate wall and the second curved member.

In the illustrative embodiment, a mass transfer device is provided in which the device has a housing enclosing a mass transfer medium and a spaced heat exchanger. The mass transfer medium is operative to enable mass transfer between a first fluid, such as blood, and a third fluid, such as oxygen. The mass transfer device includes the aforesaid heat exchanger and means are provided for conveying the first fluid, such as blood, that has been through the heat exchanger, from the heat exchanger to one area of the mass transfer medium. The third fluid, such as oxygen, is introduced to another area of the mass transfer medium to enable mass transfer to occur.

In one embodiment of the invention, means are provided in the manifold for creating turbulent flow of the second fluid as it exits from the first chamber. In an illustrative embodiment, the first member and the second member define openings between the first chamber and the second chamber. A screen member is located between the first chamber and the second chamber.

In accordance with the present invention, a process is provided for making a heat exchanger. The process comprises the steps of providing a flexible metal hose, annealing the flexible metal hose, thereafter compressing the annealed hose, and thereafter expanding the compressed hose to a length that is substantially less than its original length but greater than its length during compression.

A more detailed explanation of the invention is provided in the following description and claims, and is illustrated in the accompanying drawings.

Brief description of the drawings

Figure 1 is a perspective view, partially broken for clarity, of a mass transfer device constructed in accordance with the principles of the present invention.

Figure 2 is an exploded perspective view of the heat exchanger used in the mass transfer device of Figure 1.

Figure 3 is a perspective diagrammatic view of a fluid manifold used in the heat exchanger of Figure 2.

Figure 4 is an elevational view, partially shown in phantom for simplicity, of a heat exchanger constructed in accordance with the principles of the present invention.

Figure 5 is a cross-sectional elevation of a slightly modified mass transfer device.

Figure 6 is a cross-sectional view of the mass transfer device of Figure 5, taken along the plane of the line 6—6 of Figure 5.

Figure 7 is a flow chart of the steps comprising a process of making a heat exchanger core.

Figure 8a is a diagrammatic view of a cross-section of a high pressure flexible hose, without showing the metal thickness.

Figure 8b is a diagrammatic view of a cross-section of a heat exchanger core that has been constructed from the Figure 8a hose, with the bottom portion of Figure 8b being a greatly enlarged section of the top portion of Figure 8b.

Figure 9 is an end view of a fluid manifold for the heat exchanger of the present invention, according to another form of the invention, and showing with arrowed lines the fluid flow.

Figure 10 is a perspective view of the manifold of Figure 9.

Figure 11 is a perspective view of an alternative manifold.

Detailed description of the illustrative embodiment

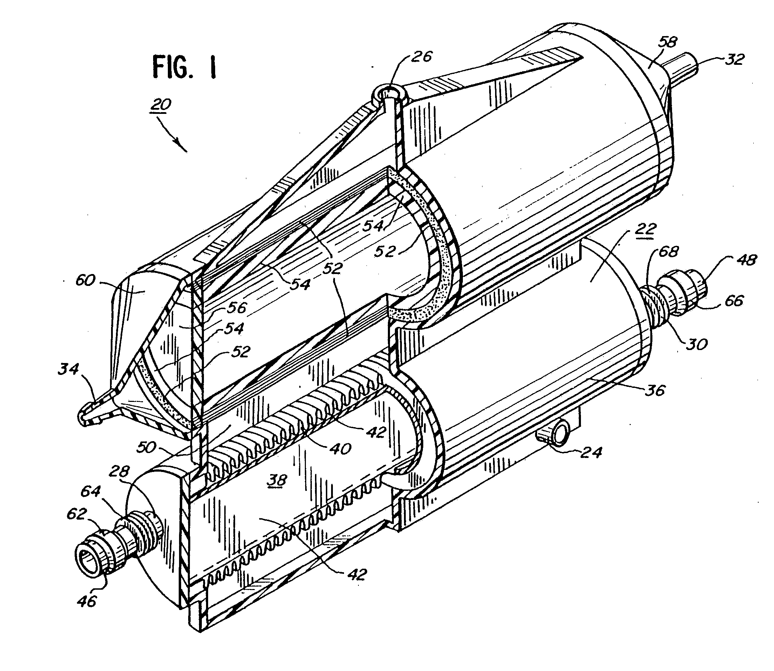

Referring to the drawings, a heat exchanger constructed in accordance with the principles of the present invention is disclosed therein, used in a blood oxygenator environment. In the perspective view of Figure 1, partially broken for clarity, oxygenator 20 includes a molded plastic housing 22 with a first fluid inlet 24 for use as a blood inlet, a first fluid outlet 26 for use as a blood outlet, a second fluid inlet 28 which operates as a water inlet, a second fluid outlet 30 which operates as a water outlet, a third fluid inlet 32 which operates as an oxygen inlet, and a third fluid outlet 34 which operates as an oxygen outlet. Housing 22 has a lower portion 36 which encloses the heat exchanger 38.

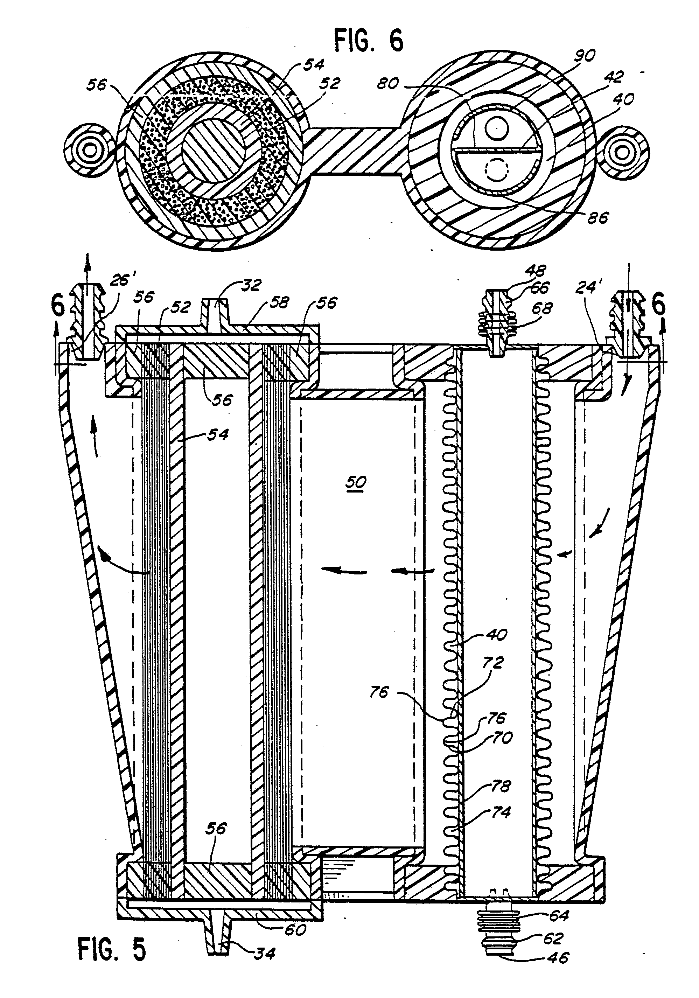

Referring to Figures 1—6, heat exchanger 38 includes a core 40, a manifold 42, end caps 44, inlet port 46 and outlet port 48. In Figures 5 and 6, an oxygenator 20' is illustrated. Oxygenator 20' is similar to oxygenator 20 of Figure 1, but the blood inlet 24' and the blood outlet 26' are at the top of the oxygenator instead of being at the side of the oxygenator as in Figure 1.

As illustrated most clearly in Figures 1 and 5, a passageway 50 couples the heat exchanger 38 with the outside of a mass transfer medium 52. In the illustrative embodiment, mass transfer medium 52 comprises a hollow fiber bundle in tubular form as is well known in the art, wound about a plastic core 54. The hollow fiber bundle 52 is encapsulated at its ends by means of a potting compound 56, although the ends of the hollow fibers are open to form oxygen flow paths as is known in the art. Housing end caps 58 and 60 secure the ends of the mass transfer device assembly.

The blood path is into inlet 24, around the outside of heat exchange 38, then around the hollow fiber bundle 52 and out of outlet 26. The cooling and/or heating water flows via inlet port 46 into inlet 28, through manifold 42 and inside the heat exchanger core 40, and out outlet 30 and outlet port 48. The oxygen flows into inlet 32, the fiber bundle and out the outlet 34.

To enable the inlet water hose (not shown) and the outlet water hose (not shown) to be connected to inlet port 46 and outlet port 48, respectively, more effectively, and to alleviate spillage, a snap-on coupling 62 and a corrugated portion 64 is formed with inlet port 46. Snap-on portion 62 enables the water hose to be snapped onto inlet port 46 and corrugations 64 enable the inlet port 46 to be bent for ease in connection and handling. Likewise, outlet port 48 is formed with snap-on portion 66 and with corrugations 68 to enable an outlet hose to be quick-coupled to outlet port 48 and to enable the outlet port 48 to bend for ease in connection and handling.

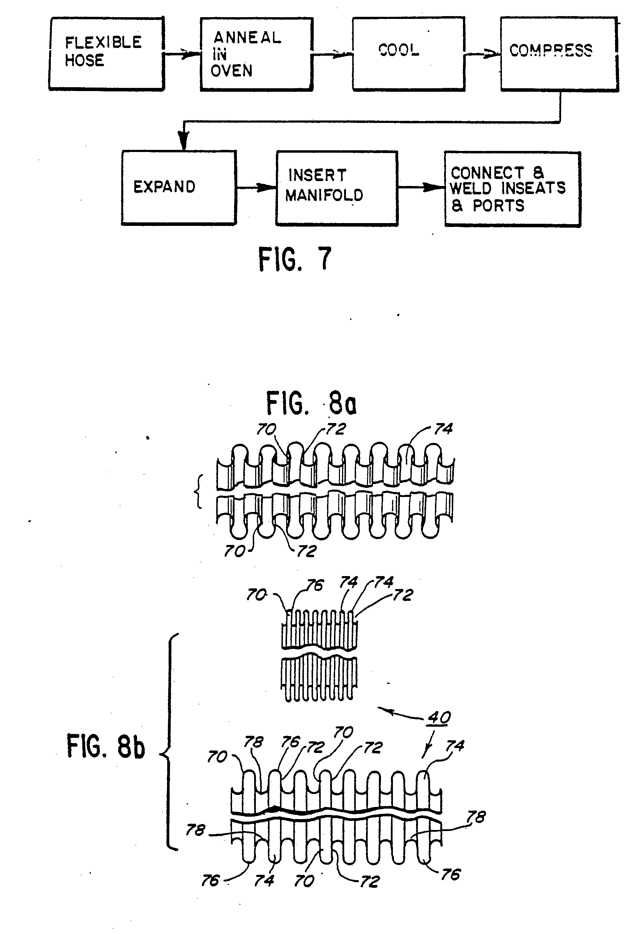

The heat exchanger core 40 of the present invention is novel and has been found to be effective to enable substantially uniform flow of the fluids that will be in heat exchange relationship with each other. A construction of heat exchanger core 40 and its method of manufacture are explained as follows, with particular reference to Figures 7—8.

The heat exchanger core 40 is formed from a high pressure flexible metal hose, of the type that is often sold in auto parts stores for use as the flexible metal hose for conveying cooling fluid within the engine compartment. In the illustrative embodiment, the outer diameter of the flexible hose is 2 inches (50.8 mm) and the inner diameter is 1.5 inches (38.1 mm). Typically, this flexible metal hose is corrugated, with approximately six corrugations per inch (25.4 mm).

A broken, enlarged diagrammatic view of portion of this flexible metal hose is presented in Figure 8a. Referring to Figure 8a, it can be seen that the side walls 70, 72 of each corrugation are not parallel to each other. It should be noted that Figures 8a to 8b are not to scale and are diagrammatic only.

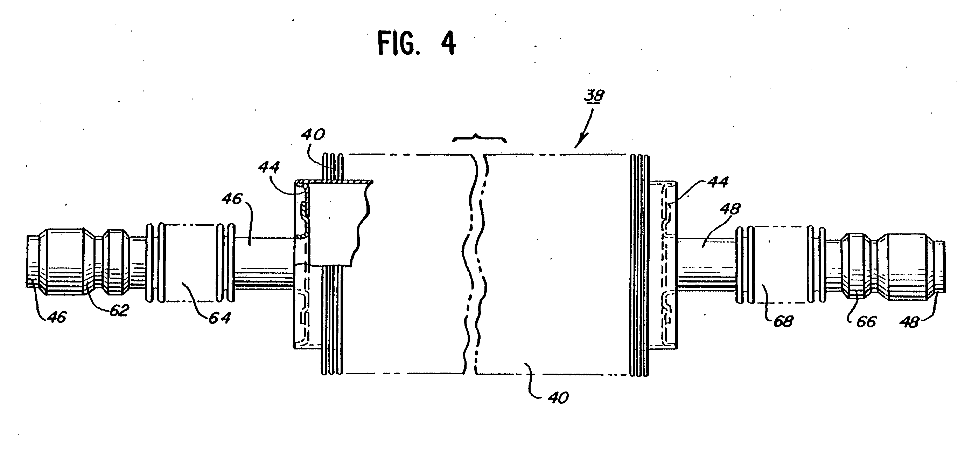

The steps for forming heat exchanger core 40 are illustrated in Figure 7. The flexible hose as described above is provided, and it is annealed in an oven. The annealed hose is allowed to cool and is then compressed to between 5 percent and 25 percent of its original length, preferably about 20 percent of its original length. Thereafter the opposite ends are pulled apart so that it is expanded to 1.5 times to 3 times its compressed length, preferably about 2 times its compressed length. Manifold 42 is then inserted into the heat exchanger core and thereafter the end caps and ports are connected and welded to form the resulting heat exchanger illustrated in Figure 4.

As the flexible metal hose is compressed, side walls 70, 72 are still curvilinear and are not parallel to each other. This will prevent the desirable uniform flow because the flow will favor the more open areas and there will be unsuitable flow in the relatively closed areas. The flexible hose is compressed to about 20 percent of its original length, but flow becomes even more restricted because of the hairpin-type configuration. However, we have discovered that after compression, when the flexible hose is expanded to 1.5 times to 3 times its compressed length, preferably 2 times its compressed length, the side walls 70, 72 become substantially parallel with each other, providing more constant volume with substantially uniform flow. Referring to Figure 8b, it can be seen that the resulting heat exchanger core comprises a plurality of contiguous corrugations 74, with each of the corrugations having an outer wall 76, a contiguous first side wall 70, a contiguous inner wall 78, and a contiguous second side wall 72, with the first side wall 70 and the second side wall 72 being substantially parallel to each other.

In the illustrative embodiment, the length of the original flexible hose was 22 inches (559 mm) and it had approximately 6 corrugations per inch (25.4 mm). This flexible metal hose is formed of a noncorrosive substance such as stainless steel or aluminum having a high thermal conductivity, and it was subjected to annealing in an oven at approximately 1000°F (538°C). The annealed hose was then removed from the oven, air cooled and then compressed to a length of 4 inches (101.6 mm), in which it had approximately 33 corrugations per inch (25.4 mm). The compressed metal hose was then expanded so that its length became 7.5 inches (190.5 mm) having approximately 18 corrugations per inch (25.4 mm).

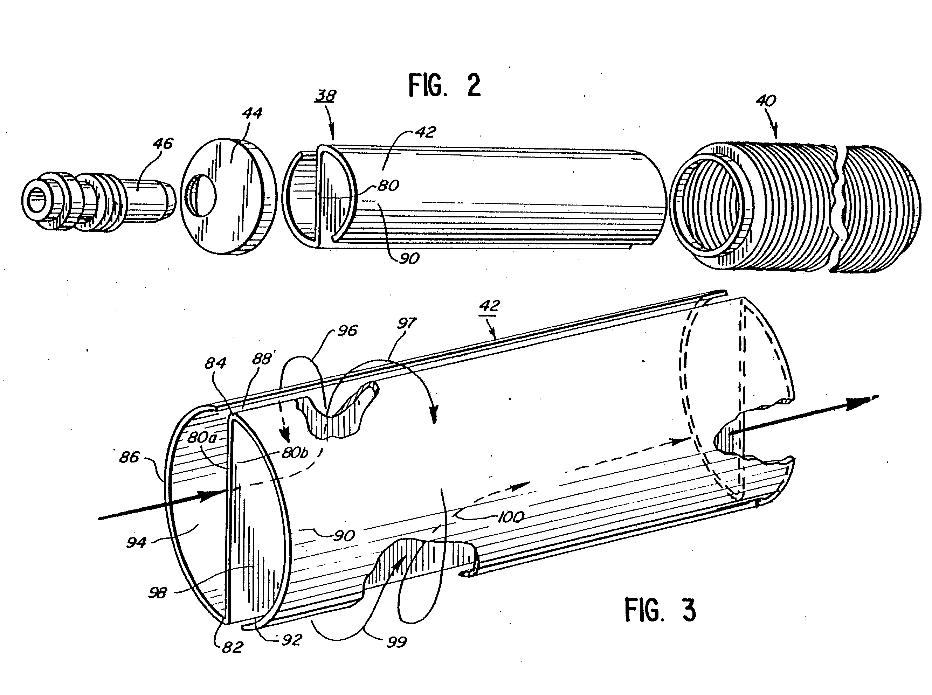

Referring to Figures 2 and 3, the manifold 42 which is inserted into heat exchanger core 40 is illustrated therein. Manifold 42 operates to distribute the second fluid which flows from inlet 28 to outlet 30 (Figure 1). Manifold 42 has a generally S-shaped cross sectional configuration, with the S-shape being defined by an intermediate wall 80 having opposed ends 82, 84. A first curved member 86 extends from end 82 and toward the other end 84 but spaced therefrom to form a first longitudinal slot 88. A second curved member 90 extends from end 84 and toward end 82 but is spaced therefrom to form a second longitudinal slot 92. The second fluid, i.e., water, is introduced into a first chamber 94 defined by one side 80a of intermediate wall 80 and the first curved member 94. The first fluid will flow in chamber 94, out of slot 88, around curved members 86 and 90 in the direction of arrows 96, 97, through slot 92 and into chamber 98 in the direction of arrows 99, 100, through chamber 98 and out of chamber 98. Chamber 98 is defined by side 80b of intermediate member 80 and curved member 90.

It can be seen that the cooling or heating water will flow on the inside of heat exchager core 40 and the blood will flow on the outside thereof. The blood that has been heat exchanged will then flow into the mass transfer area where it will be oxygengated with the oxygenated blood exiting via blood outlet 26.

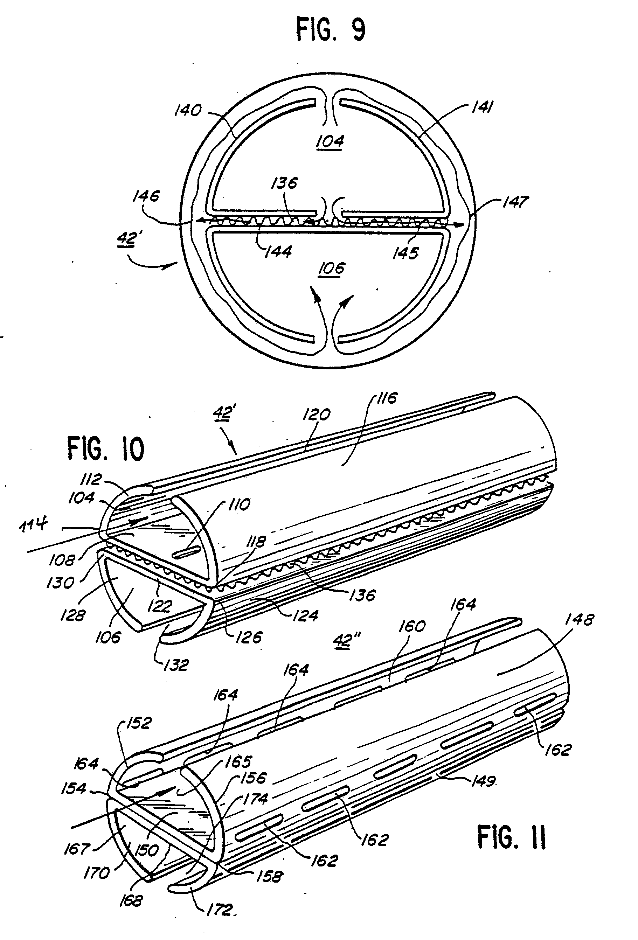

In another form of the invention, device for providing turbulent flow is utilized. Heat transfer in turbulent flow is significantly greater than heat transfer in laminar fluid flow, due to the turbulence induced mixing. To this end, a turbulence inducer is illustrated in Figures 9—11, which turbulence inducer is intended to achieve greater overall heat transfer.

Referring to Figures 9 and 10, a manifold 42' is shown therein comprising a first chamber 104 and a second chamber 106. The first chamber is defined by a first member having a planar base 108 defining a series of collinear spaced slots 110. Although not all of the slots are illustrated, in the embodiment of Figures 9 and 10, there may be between four and ten slots 110. A first curved member 112 extends from end 114 of base 108 and a second curved member 116 extends from end 118 of base 108. Curved members 112 and 116 extend toward each other but are spaced from each other to define a longitudinal slot 120.

The second chamber 106 is defined by a second base member 122 having a third curved member 124 extending from end 126 thereof. A fourth curved member 128 extends from end 130 of base 122, with third and fourth curved members 124 and 128 extending toward each other, but being spaced from each other to define a longitudinal slot 132. Slot 132 is on the opposite side of manifold 42' from slot 120, and the cross-sectional configuration of curved members 112, 116, 124 and 128 is substantially circular. A screen 136 is interposed between base 108 and base 122 to aid in creating the turbulent flow desired. Manifold 42' is inserted into heat exchanger core 40, in the same manner that manifold 42 (Figure 2) is inserted into heat exchanger core 40. Water inlet 28 (Figure 1) will introduce the entry water to chamber 104 and most of the water will flow via slot 120, around the outside of the manifold (i.e., inside the heat exchanger core) in the direction of arrowed lines 140, 141, through slot 132 and into chamber 106, and out the end of chamber 106 opposite to the entry end of chamber 104. Some of the water will flow through slots 110 and pass through screen 136 in the direction of arrowed flow lines 144, 145. This water will enter the flow at about points 146 and 147 (Figure 9) to enter the flow stream at substantially a right angle, to create turbulence.

In Figure 11, a manifold 42″ is illustrated, and it includes an upper member 148 comprising a base 150, a first curved member 152 extending from one end 154 of base 150 and a second curved member 156 extending from end 158 of base 150. Curved members 152 and 156 curve toward each other but are spaced to form a longitudinal slot 160. As illustrated, a series of collinear slots 162 are defined by second curved member 156 toward the junction between curved member 156 and base 150. Likewise, first curved member 152 defines a plurality of collinear slots 164 adjacent the junction between first curved member 152 and base 150. The upper member 148 illustrated in Figure 11 defines a first chamber 165. A second chamber 167 is defined by a connected lower member 149 including base 168, third curved member 170 and fourth curved member 172. Curved members 170 and 172 curve toward each other and are spaced to form a slot 174.

The water will flow into the first chamber 165 defined by the upper member 148 with some of the water flowing up through slot 160 and around the outside of the manifold, then back into slot 174 and out the second chamber 167. Some of the water from the first chamber 165 will flow through slots 162 and 164 to enter the outside flow at an angle to the flow stream to create turbulence.

A disposable blood oxygenator has been disclosed that is relatively compact, has a low prime volume, is easy to manufacture and has a relatively low cost. The heat exchanger in accordance with the present invention enables substantially uniform flow of the fluid that will be in heat exchange relationship with each other.

**Claims**

1. A heat exchanger which comprises:
a core (40) having an inside and an outside and formed of a corrugated metal having a high thermal conductivity, each of the corrugations (74) of the core comprising an outer wall (76), a contiguous first side wall (70), a contiguous inner wall (78), and a contiguous second side wall (72), with the first and second side walls being substantially parallel to each other, and with a plurality of said corrugations repeating contiguously;
means (24) for introducing a first fluid onto the outside of the core and means (28) for introducing a second fluid into the inside of the core, whereby the first and second fluids will be in heat exchange relationship with each other and the flow of the fluids is substantially uniform resulting from the substantially parallel first and second side walls of the corrugations; characterized by
a manifold (42) for the second fluid located within the inside of the core, the fluid manifold defining a first longitudinal slot (88) open to the outside of the fluid manifold and a second longitudinal slot (92) open to the outside of the fluid manifold, with said first and second slots being on opposed sides of the manifold, said manifold (42) having an intermediate wall (80) which defines a first chamber (94) communicating with the first slot and a second chamber (98) communicating with the second slot, the fluid manifold being so configured that the second fluid may move in said first chamber, through said first slot, around the outside of the fluid manifold into said second slot and out from said second chamber.

2. A heat exchanger according to Claim 1 wherein the manifold has a generally S-shaped cross-sectional configuration.

3. A heat exchanger according to Claim 2, wherein the S-shape is defined by the intermediate wall (80) having opposed ends (82, 84) with a first curved member (86) extending from one end (82) and towards the other end (84) but spaced therefrom to form the first slot (88), and with a second curved member (90) extending from the other end (84) and towards said one end (82) but spaced therefrom to form the second slot (92); the first chamber (94) being defined by one side of said intermediate wall and said first curved member and the second chamber (98) being defined by the opposite side of said intermediate wall and said curved member.

4. A heat exchanger according to Claim 1, wherein the manifold comprises means for creating turbulent flow of the second fluid as it exits from the first chamber.

5. A heat exchanger according to Claim 4, including a screen member located between the first chamber and the second chamber.

6. A mass transfer device having a housing (22) which encloses a mass transfer medium (52) and a spaced heat exchanger (38) according to any preceding claim, with the mass transfer medium being operative to enable mass transfer between the first fluid and a third fluid, the device including means for conveying the first fluid that has been heat exchanged from the heat exchanger to one area of the mass transfer medium; and means for introducing said third fluid to another area of the mass transfer medium to enable mass transfer to occur.

7. A process for making a heat exchanger according to any one of Claims 1 to 5, comprising the step of:
providing a flexible metal hose;
annealing the flexible metal hose;
thereafter compressing the annealed hose; and
thereafter expanding the compressed hose to a length that is substantially less than its original length but greater than its length during compression.

8. A process according to Claim 7, including the step of cooling the annealed hose prior to compression.

9. A process according to Claim 7 or 8, wherein the hose is compressed to between 5 percent and 20 percent of its original length and is expanded to about 1.5 times to about 3 times its compressed length.

10. A process according to Claim 9, in which the hose is compressed to about 20 percent of its original length and is expanded to about 2 times its compressed length.

## Patentansprüche

1. Wärmetauscher, der folgendes aufweist:
einen Kern (40), der eine Innenseite und eine Außenseite hat und der aus einem gewellten Metall mit einer hohen Wärmeleitfähigkeit gebildet ist, wobei jede der Wellen (74) des Kernes eine Außenwand (76), eine zusammenhängende erste Seitenwand (70), eine zusammenhängende Innenwand (78) und eine zusammenhängende zweite Seitenwand (72) hat, wobei die ersten und zweiten Seitenwände im wesentlichen parallel zueinander verlaufen, und wobei eine Vielzahl der Wellen sich zusammenhängend wiederholt;
eine Einrichtung (24) zum Einleiten eines ersten Fluids auf die Außenseite des Kernes und eine Einrichtung (28) zum Einleiten eines zweiten Fluids in den Innenraum dem Kernes, so daß die ersten und zweiten Fluide in eine Wärme tauschende Relation zueinander Kommen und die Strömung der Fluide im·wesentlichen gleichförmig ist, was aus den im wesentlichen parallelen ersten und zweiten Seitenwänden der Wellenkonfiguration oder Rillenbildung resultiert, gekennzeichnet durch
einen Verteiler (42) für das zweite Fluid, das sich innerhalb des Innenraumes des Kernes befindet, wobei der Fluidverteiler einen ersten Längsschlitz (88), der zur Außenseite des Fluidverteiles offen ist, und einen zweiten Längsschlitz (92) bildet, der zur Außenseite des Fluidverteilers offen ist, wobei die ersten und zweiten Schlitze sich auf gegenüberliegenden Seiten des Verteilers befinden, wobei der Verteiler (42) eine Zwischenwand (80) aufweist, die eine erste Kammer (94) in Verbindung mit dem ersten Schlitz und eine zweite Kammer (98) in Verbindung mit dem zweiten Schlitz bildet, wobei der Fluidverteiler eine solche Konfiguration hat, daß das zweite Fluid sich in der ersten Kammer, durch den ersten Schlitz, um die Außenseite des Fluidverteilers in den zweiten Schlitz hinein und aus der zweiten Kammer hinaus bewegen kann.

2. Wärmetauscher nach Anspruch 1, wobei der Verteiler eine im allgemeinen S-förmige Querschnittskonfiguration hat.

3. Wärmetaucher nach Anspruch 2, wobei die S-Form von der Zwischenwand (80), die gegenüberliegende Enden (82, 84) hat, mit einem ersten gebogenden Teil (86), das sich von dem einen Ende (82) aus zu dem anderen Ende (84) erstreckt, aber von diesem zur Bildung des ersten Schlitzes (88) beabstandet ist, und mit einem zweiten gebogenen Teil (90) gebildet wird, das sich von dem anderen Ende (84) zu dem einen Ende (82) erstreckt, aber von diesem zur Bildung des zweiten Schlitzes (92) beabstandet ist; wobei die erste Kammer (94) von der einen Seite der Zwischenwand und dem ersten gebogenen Teil gebildet wird, während die zweite Kammer (98) von der gegenüberliegenden Seite der Zwischenwand und dem gebogenen Teil gebildet wird.

4. Wärmetauscher nach Anspruch 1, wobei der Verteiler eine Einrichtung aufweist, um eine turbulente Strömung des zweiten Fluids zu erzeugen, wenn es aus der ersten Kammer austritt.

5. Wärmetauscher nach Anspruch 4, enthaltend ein Siebteil, das zwischen der ersten Kammer und der zweiten Kammer angeordnet ist.

6. Massenübertragungseinrichtung, mit einem Gehäuse (22), das ein Massenübertragungsmedium (52) und einen beabstandeten Wärmetauscher (38) nach einem der vorhergehenden Ansprüche umschließt, wobei das Massenübertragungsmedium so wirkt, daß es eine Massenübertragung zwischen dem ersten Fluid und einem dritten Fluid ermöglicht, wobei die Einrichtung Mittel enthält, um das erste Fluid, das einem Wärmeaustausch unterzogen worden ist, von dem Wärmetauscher zu einem Bereich des Massenübertragungsmediums zu überführen, sowie Mittel aufweist, um das dritte Fluid in einen anderen Bereich des Massenübertragungsmediums einzuleiten, damit eine Massenübertragung stattfinden kann.

7. Verfahren zur Herstellung eines Wärmetauschers nach einem der Ansprüche 1 bis 5, umfassend die folgenden Schritte:
Herstellen eines flexiblen Metallschlauches;
Glühen des flexiblen Metallschauches;
anschließendes Komprimieren des gekühlten Schlauches; und
anschließendes Expandieren des komprimierten Schlauches auf eine Länge, die wesentlich kleiner ist als seine ursprüngliche Länge, aber größer als seine Länge während der Kompression.

8. Verfahren nach Anspruch 7, umfassend den Schritt des Kühlens des geglühten Schlauches vor der Kompression.

9. Verfahren nach Anspruch 7 oder 8, wobei der Schlauch auf einen Wert zwischen 5% und 20% seiner ursprünglichen Länge komprimiert und auf das 1,5-fache bis etwa 3-fache seiner komprimierten Länge gedehnt wird.

10. Verfahren nach Anspruch 9, wobei der Schlauch auf etwa 20% seiner ursprünglichen Länge komprimiert und auf etwa das 2-fache seiner komprimierten Länge gedehnt wird.

## Revendications

1. Echangeur de chaleur qui comprend:
une âme (40) présentant un côté intérieur et un côté extérieur et qui est fabriquée en un métal ondulé de conductivité thermique élevée, chacune des ondulations (74) de l'âme comprenant une paroi extérieure (76), une première paroi latérale contigué (70), une paroi inférieure contigué (78) et une deuxième paroi latérale contigué (72), les premières et deuxième parois latérales étant sensiblement parallèles l'une à l'autre et une pluralité desdites ondulations se répétant en contiguïté;

des moyens (24) pour introduire un premier fluide du côté extérieur de l'âme et des moyens (28) pour introduire un deuxième fluide du côté intérieur de l'âme, de sorte que les premier et deuxième fluides sont en relation mutuelle d'échange de chaleur et l'écoulement des fluides est sensiblement uniforme du fait que les première et deuxième parois latérales des ondulations sont sensiblement parallèles,

caractérisé en ce que un répartiteur (42) pour le deuxième fluide est disposé à l'intérieur de l'âme, le répartiteur de fluide comportant une première fente longitudinale (88) qui débouche à l'extérieur du répartiteur de fluide et une deuxième fente longitudinale (92) qui débouche à l'extérieur du répartiteur de fluide, lesdites première et deuxième fentes étant situées sur des côtés opposés du répartiteur, ledit répartiteur (42) comportant une paroi intermédiaire (80) qui définit une première chambre (94) en communication avec la première fente et une deuxième chambre (98) en communication avec la deuxième fente, le répartiteur de fluide ayant une configuration telle que le deuxième fluide peut circuler dans ladite première chambre, à travers ladite première fente, autour de l'extérieur du répartiteur de fluide, pénétrer dans ladite deuxième fente et sortir de ladite deuxième chambre.

2. Echangeur de chaleur suivant la revendication 1, dans lequel le répartiteur à une configuration de section transversale sensiblement en forme de S.

3. Echangeur de chaleur suivant la revendication 2, dans lequel la forme en S est définie par la paroi intermédiaire (80) ayant des extrémités opposées (82, 84), avec une première partie courbe (86) qui s'étend à partir d'une première extrémité (82) et vers l'autre extrémité (84) mais qui est espacée de cette dernière pour former la première fente (88), et avec une deuxième partie courbe (90) qui s'étend à partir de l'autre extrémité (84) et vers ladite première extrémité (82) mais qui est espacée de cette dernière pour former la deuxième fente (92), la première chambre (94) étant définie par une face de ladite paroi intermédiaire et ladite première partie courbe et la deuxième chambre (98) étant définie par la face opposée de ladite paroi intermédiaire et ladite partie courbe.

4. Echangeur de chaleur suivant la revendication 1, dans lequel le répartiteur comprend des moyens pour engendrer un écoulement turbulent du deuxième fluide à sa sortie de la première chambre.

5. Echangeur de chaleur suivant la revendication 4, comprenant un écran placé entre la première chambre et la deuxième chambre.

6. Dispositif de transfert de masse comprenant une enveloppe (22) qui entoure un milieu de transfert de masse (52) et un échangeur de chaleur espacé (38) suivant l'une quelconque des revendications précédentes, le milieu de transfert de masse agissant pour permettre un transfert de masse entre le premier fluide et un troisième fluide, le dispositif comprenant des moyens pour diriger le premier fluide qui a échangé de la chaleur avec l'échangeur de chaleur jusqu'à une zone de transfert de masse, et des moyens pour introduire ledit troisième fluide dans une autre zone du milieu de transfert de masse afin que le transfert de masse puisse se produire.

7. Procédé de fabrication d'un échangeur de chaleur suivant l'une quelconque des revendications 1 à 5, comprenant les opérations de:
préparation d'un tuyau métallique flexible;
recuit du tuyau métallique flexible;
ensuite compression du tuyau recuit; et
ensuite allongement du tuyau comprimé, jusqu'à une longueur qui est sensiblement inférieure à sa longueur initiale mais supérieure à sa longueur pendant la compression.

8. Procédé suivant la revendication 7, comprenant l'opération de refroidissement du tuyau recuit, avant la compression.

9. Procédé suivant la revendication 7 ou 8, dans lequel le tuyau est comprimé à une valeur comprise entre 5% et 20% de sa longueur initiale et il est allongé à une valeur comprise entre 1,5 fois environ et 3 fois environ sa longueur comprimée.

10. Procédé suivant la revendication 9, dans lequel le tuyau est comprimé à 20% environ de sa longueur initiale et il est allongé à deux fois environ sa longueur comprimée.

FIG. 1

20

26
58
32
54
52
52
54
52
54
60
56
54
52
34
22
68
48
66
30
36
50
42
40
38
42
24
28
64
62
46

0 174 319

FIG. 2

FIG. 3

FIG. 4

# FIG. 6

# FIG. 5

FIG. 7

FIG. 8a

FIG. 8b

# FIG. 9

# FIG. 10

# FIG. 11